(19) 

Europäisches
Patentamt
European
Patent Office
Office européen
des brevets

(11) **EP 1 767 203 A1**

(12) **EUROPEAN PATENT APPLICATION**
published in accordance with Art. 158(3) EPC

(43) Date of publication:
**28.03.2007 Bulletin 2007/13**

(21) Application number: **05765407.1**

(22) Date of filing: **29.06.2005**

(51) Int Cl.:
***A61K 31/352*** *(2006.01)*     ***A23F 3/14*** *(2006.01)*
***A23L 1/30*** *(2006.01)*

(86) International application number:
**PCT/JP2005/011904**

(87) International publication number:
**WO 2006/003909 (12.01.2006 Gazette 2006/02)**

(84) Designated Contracting States:
**AT BE BG CH CY CZ DE DK EE ES FI FR GB GR
HU IE IS IT LI LT LU MC NL PL PT RO SE SI SK TR**

(30) Priority: **30.06.2004 JP 2004194696**

(71) Applicant: **SUNTORY LIMITED
Osaka-shi,
Osaka 530-8203 (JP)**

(72) Inventors:
- **TANAKA, Hiroaki
  Fukuoka-shi,
  Fukuoka 8140155 (JP)**
- **KISO, Yoshinobu
  5670872 (JP)**
- **IINO, Taeko
  5200113 (JP)**

(74) Representative: **Vossius & Partner
Siebertstrasse 4
81675 München (DE)**

(54)  **FAT COMBUSTION ACCELERATOR**

(57)    The present invention provides a novel fat combustion accelerator which can promote lipid metabolism without changing the total consumption calories, **characterized in that** said fat combustion accelerator comprises proanthocyanidin as an active ingredient.

Fig. 1

**Description**

Technical Field

The present invention relates to a fat combustion accelerator, a body fat reducer, an adiposity preventing agent, and a drink or food for fat combustion acceleration, body fat reduction or adiposity prevention, which are useful for maintaining health.

Background Art

Proanthocyanidin is a kind of polyphenols contained in plants, and has been known to have a variety of activities such as antioxidation and the like (see JP-B-1991-7232, Bagchi, D. et al, Toxicology, 2000, Vol.148, p.187-189, and Fremont L. et al, Life Sciences, 1999, Vol.64, p.2511-2521). With regard to the action of proanthocyanidin, an activity of elimination of superoxide radicals has been known, and its synergistic action with vitamin C having an antioxidation action has been reported (see Bagchi, D. et al , Toxicology, 2000, Vol . 148 , p.187-189). Moreover, with regard to a clinical action, its therapeutic effect against chronic pancreatitis has also been known (see Bagchi, D. et al, Toxicology, 2000, Vol. 148, p. 187-189, andFremont L. et al, Life Sciences, 1999, Vol.64, p.2511-2521).

Disclosure of the Invention

An object of the present invention is to provide a novel fat combustion accelerator, a body fat reducer, an adiposity preventing agent, and a drink or food for fat combustion acceleration, body fat reduction or adiposity prevention, permitting acceleration of fat combustion without changing the total consumption calories.

As a result of having diligently studied for achieving the above-described purposes, the present inventors have found that there is no difference in the total consumption calories between the cases where proanthocyanidin was administered and where proanthocyanidin was not administered. However, in the case where proanthocyanidin was administered, fat metabolism is enhanced, that is, fat is more efficiently consumed. Based on these findings, the present inventors have found that proanthocyanidin is useful as a fat combustion accelerator, a body fat reducer, and an adiposity preventing agent.

Moreover, the present inventors have completed the present invention by further studying after obtaining such various findings. Thus, the present invention relates to the followings:

(1) a fat combustion accelerator, which comprises proanthocyanidin as an active ingredient,
(2) the fat combustion accelerator described in the above (1), wherein proanthocyanidin is an extract derived from pine bark,
(3) the fat combustion accelerator described in the above (1) or (2), wherein proanthocyanidin is an oligomeric proanthocyanidin,
(4) the fat combustion accelerator described in any one of the above (1) to(3), which is in the form of tablet, pill, capsule, granule, powder, pulvis, or liquid and solution,
(5) a body fat reducer, which comprises proanthocyanidin as an active ingredient,
(6) the body fat reducer described in the above (5), wherein proanthocyanidin is an extract derived from pine bark,
(7) the body fat reducer described in the above (5) or (6),
wherein proanthocyanidin is an oligomeric proanthocyanidin,
(8) the body fat reducer described in any one of the above (5) to (7), which is in the form of tablet, pill, capsule, granule, powder, pulvis, or liquid and solution,
(9) an adiposity preventing agent, which comprises proanthocyanidin as an active ingredient,
(10) the adiposity preventing agent described in the above (9), wherein proanthocyanidin is an extract derived from pine bark,
(11) the adiposity preventing agent described in the above (9) or (10), wherein proanthocyanidin is an oligomeric proanthocyanidin,
(12) the adiposity preventing agent described in any one of the above (9) to (11), which is in the form of tablet, pill, capsule, granule, powder, pulvis, or liquid and solution,
(13) a drink or food, which comprises the fat combustion accelerator described in any one of the above (1) to (4),
(14) a drink or food, which comprises the body fat reducer described in any one of the above (5) to (8),
(15) a drink or food, which comprises the adiposity preventing agent described in any one of the above (9) to (12),
(16) a drink or food for fat combustion acceleration, body fat reduction or adiposity prevention, which comprises proanthocyanidin as an active ingredient,
(17) the drink or food described in the above (16), which is a drink or food for body fat combustion acceleration,

(18) the drink or food described in the above (16), which is a drink or food for body fat reduction,

(19) the drink or food described in the above (16), which is a drink or food for body adiposity prevention,

(20) the drink or food described in any one of the above (16) to (19), wherein proanthocyanidin is an extract derived from pine bark,

(21) the drink or food described in any one of the above (16) to (20), wherein proanthocyanidin is an oligomeric proanthocyanidin,

(22) the drink or food described in any one of the above (16) to (21), which is a solid food, a gel food, or a drink, and

(23) the drink described in any one of the above (16) to (22), which is a soft drink or a tea drink.

Effect of the Invention

A fat combustion accelerator, a body fat reducer, an adiposity preventing agent, and a drink or food for fat combustion acceleration, body fat reduction or adiposity prevention of the present invention are useful for maintaining health, and exert the effect of promoting lipid metabolism without changing the total consumption calories.

Brief Description of the Drawing

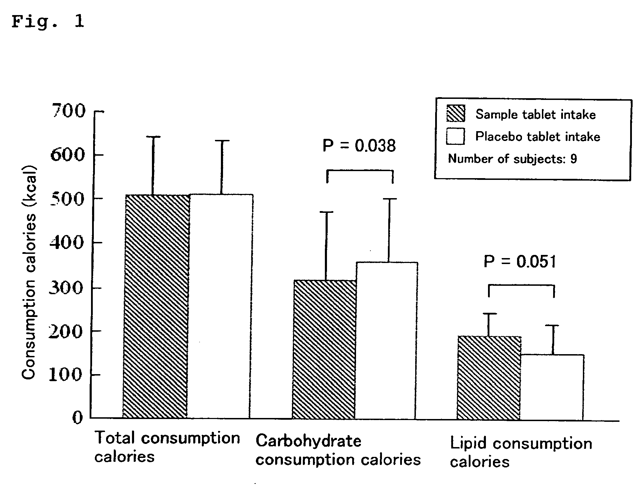

Fig. 1 is a diagram showing the calculation results of the total consumption calories, carbohydrate consumption calories and lipid consumption calories in Test Example 1.

Best Mode For Carrying Out the Invention

A fat combustion accelerator, a body fat reducer, an adiposity preventing agent, and a drink or food for fat combustion acceleration, body fat reduction or adiposity prevention of the present invention (hereinafter, these are also referred to collectively as "fat combustion accelerator and the like") are characterized in that these comprises proanthocyanidin as an active ingredient.

Proanthocyanidin used as an active ingredient such as a fat combustion accelerator and the like of the present invention is referred to a group of compounds, their derivatives and stereoisomers thereof, comprising condensation polymers having a constitution unit of flavan-3-ol and/or flavan-3,4-diol, and having a polymerization degree of dimer or more, preferably dimer to decamer, and more preferably dimer to tetramer. Among proanthocyanidins, a condensation polymer having a polymerization degree of dimer to tetramer wherein flavan-3-ol and/or flavan-3,4-diol are/is constitution unit (s) is referred to OPC (oligomeric proanthocyanidin). OPC is a strong antioxidant substance (see JP-B-19 91-72 32), and it is abundantly contained in leaves and barks of plants, and rinds or seeds of fruits. To be more specific, OPC is contained in grape, pine bark, inner skin of peanut, ginkgo, fruit of false acacia, cowberry, blueberry, strawberry, avocado, barley, wheat, soybean, black soybean, cacao and the like. It has also been known that OPC is contained in cola nuts of West Africa, and root of Rathania of Peru. OPC is a substance which is not produced in human body.

Concerning with proanthocyanidin used as an active ingredient such as fat combustion accelerator and the like of the present invention, there is no limitation at all for the source or utilizing part of the raw materials , production methods, or purification methods. However, there may be used a food raw material such as a crushed product of the above-described bark, fruit or seed, and an extract thereof or the like. Particularly, it is preferable to use an extract derived from pine bark, or more preferable to use an extract derived from the bark of pine tree in the seaside of France in which OPC is abundantly contained. The pine bark obtained from the seaside of France is preferably used as a raw material of proanthocyanidin.

Proanthocyanidin can be easily obtained from the above-described various plants by employing a known method (for example, a method described in JP-B-1991-7232 or an extraction method from pine bark; R. W. Hemingway et al., Phytochemistry, 1983, Vol.22, p.275-281) or according to a similar method thereof.

Hereinafter, a method of preparing proanthocyanidin will be explained by way of an example of an extract of the pine bark in which OPC is abundantly contained.

The extract derived from the pine bark is obtained by extracting the pine bark with water or an organic solvent. In the case where water is utilized, warm water or hot water is used. As an organic solvent used for the extraction, there is used an organic solvent which is permissible for the preparation of foods or pharmaceuticals. Such organic solvents include, for example, methanol, ethanol, propanol, butanol, butane, acetone, hexane, cyclohexane, propylene glycol, hydrous ethanol, hydrous propylene glycol, ethyl methyl ketone, glycerin, methyl acetate, ethyl acetate, diethyl ether, dichloromethane, oils and fats for food, 1,1,1,2-tetrafluoroethane, 1,2-trichloroethene and the like. These water and organic solvents may be used alone or in combination thereof. Particularly, hot water, hydrous ethanol or hydrous propylene glycol is preferably used.

As for a method of extracting proanthocyanidin from the pine bark, although it is not particularly limited, for example, an extraction method under heating conditions, a supercritical fluid extraction method and the like are used.

The supercritical fluid extraction method is a method where extraction is carried out by using a supercritical fluid that is a fluid in a state of exceeding over the gas-liquid critical points (critical temperature, critical pressure) of a substance. As a supercritical fluid, there are used carbon dioxide, ethylene, propane, nitrous oxide (laughing gas) and the like are used, and particularly, carbon dioxide is preferably used.

The supercritical fluid extraction method comprises an extraction step wherein the target component is extracted with a supercritical fluid and a separation step wherein the target component and the supercritical fluid are separated. As for the separation step, any one of extraction and separation methods may be performed by changing the pressure or the temperature, or by using an adsorbent or an absorbent.

Moreover, a supercritical fluid extraction may be performed by a method of adding an entrainer. This method is a method in which, for example, ethanol, propanol, n-hexane, acetone, toluene or the like is added to a supercritical fluid in a ratio of 2 to 20%, and a supercritical fluid extraction is carried out using the resultant extraction fluid, whereby the solubility of the extraction fluid to the substance to be extracted such as OPC or the like is remarkably enhanced, or the separation selectivity is enhanced. This is a method to efficiently obtain an extract derived from the pine bark.

Except for the above-described methods, an extraction method from the pine bark may be performed by a batch method using liquid carbon dioxide, a reflux method using liquid carbon dioxide, a reflux method using supercritical carbon dioxide or the like.

Proanthocyanidin obtained above is obtained in the form of liquid or semi-solid, and if the extraction solvent is removed from the product by a known method such as evaporation in vacuo, spray-drying or freeze-drying, the residue can be used as a proanthocyanidin-containing condensate or dried composition as it is. In order to perform further purification, such purpose can be achieved by employing a known purification means such as a column chromatography, a counter-current distribution method or the like.

Proanthocyanidin contained in a fat combustion accelerator and the like of the present invention dissolves well in water, and its bioavailability is high. Under any acidic, neutral or alkaline conditions, the stability of proanthocyanidin is high, and it is easy to blend proanthocyanidin in a drink or food while maintaining its function. Moreover, since the effect of proanthocyanidin can be expected to occur within a short time after the intake, and sufficient effects can be obtained even at low doses, proanthocyanidin has a high utility value as food materials for infants or aged people who have limitation in acceptable intake and intake forms as a drink or food. Therefore, the drink or food of the present invention may be, for example, health food, food with health claims (food for specified health uses, food with nutrient function claims) and the like.

The fat combustion accelerator, the body fat reducer and the adiposity preventing agent of the present invention (hereinafter, also referred to collectively as a preparation of the present invention) can be appropriately prepared in the form of solid or liquid such as tablet, pill, capsule, granule, powder, pulvis, and liquid and solution, and the like by a known method. Thus, a solid or liquid preparation useful as a preparation of the present invention is produced by mixing proanthocyanidin with an optional additive, followed by utilizing the conventionally and sufficiently established known method for the preparation. Such additive includes, for example, excipients, pH adjusting agents, refreshing agents, suspending agents, diluents,antifoaming agents, thickeners, solubilizers, disintegrating agents, binders, lubricants, antioxidants, coating agents, coloring agents, flavoring agents, surfactants, plasticizers, fragrances and the like.

The above-described excipient includes, for example, sugar alcohols such as D-sorbitol, D-mannitol, xylitol and the like, sugars such as glucose, sucrose, lactose, fructose and the like, crystalline cellulose, carmellose sodium, calcium hydrogen phosphate, wheat starch, rice starch, corn starch, potato starch, dextrin, $\beta$-cyclodextrin, light anhydrous silicic acid, titanium oxide, magnesium aluminium metasilicate and the like.

The above-describedpH adjusting agent includes , for example, citric acid, malic acid, sodium hydrogen phosphate, dipotassium hydrogen phosphate and the like.

The above-described refreshing agent includes, for example, 1-menthol, mentha water and the like.

The above-described suspending agent includes, for example, kaolin, carmellose sodium, xanthan gum, methylcellulose and tragacanth and the like.

The above-described diluent includes, for example, purified water, ethanol, plant oil, emulsifier and the like.

The antifoaming agent includes, for example, dimethyl polysiloxane, silicon antifoaming agent and the like.

The above-described thickener includes, for example, xanthan gum, tragacanth, methylcellulose, dextrin and the like.

The above-described solubilizer includes, for example, ethanol, sucrose fatty acid ester, macrogol and the like.

The above-described disintegrating agent includes, for example, low-substituted hydroxypropyl cellulose, carboxymethylcellulose calcium, croscarmellose sodium, hydroxypropyl starch, partial $\alpha$-starch and the like.

The above-described binder includes, for example, methylcellulose, hydroxypropyl cellulose, hydroxypropylmethyl cellulose, polyvinylpyrolidone, gelatin, gum arabic, ethylcellulose, polyvinyl alcohol, pullulan, a-starch, agar, gum tragacanth, sodium alginate, propylene glycol alginate and the like.

The above-described lubricant includes, for example, stearic acid, magnesium stearate, calcium stearate, polyoxyl stearate, cetanol, talc, hydrogenated oil, sucrose fatty acid ester, dimethyl polysiloxane, beeswax, bleached beeswax and the like.

The above-described antioxidant includes, for example, ascorbic acid, dibutylhydroxytoluene (BHT), propyl gallate, butylhydroxyanisole (BHA), tocopherol, citric acid and the like.

The above-described coating agent includes, for example, hydroxypropyl methylcellulose, hydroxypropyl cellulose, methylcellulose, ethylcellulose, hydroxypropyl methylcellulose phthalate, hydroxypropyl methylcellulose acetate succinate, carboxymethylethyl cellulose, cellulose acetate phthalate, polyvinyl acetal diethylaminoacetate, aminoalkyl methacrylate copolymer, hydroxypropylmethyl cellulose acetate succinate, methacrylate copolymer, polyvinylacetate diethylaminoacetate, shellac and the like.

The above-described coloring agent includes, for example, an extract of turmeric (Curcuma longa), riboflavin, titanium oxide, carotene solution and the like.

The above-described flavoring agent includes, for example, citric acid, adipic acid, ascorbic acid, fructose, D-sorbitol, glucose, sodium saccharin, simple syrup, sucrose, honey, sweet hydrangea leaf, Glycyrrhiza glabra, orange oil, orange peel tincture, anise oil, mentha herb, menthol and the like.

The above-described surfactant includes, for example, polyoxyethylene hydrogenated castor oil, glycerine monostearate, sorbitan monostearate, sorbitan monolaurate, polyoxyetylene polyoxypropylene, polysorbates, sodium lauryl sulfate, macrogols, sucrose fatty acid ester and the like.

The above-described plasticizer, for example, triethyl citrate, polyethylene glycol, triacetin, cetanol and the like .

The above-described fragrance includes, for example, natural fragrances such as animal fragrance, plant fragrance and the like; isolated fragrances; and synthetic fragrances such as totally synthesized fragrance and the like.

With respect to the amount of proanthocyanidin in a preparation of the present invention, it is usually in the range from about 1 to 80% by weight, preferably about 2 to 50% by weight to the whole preparation.

The administration route for the preparation of the present invention may be oral or parenteral. Moreover, although the dose of proanthocyanidin as an active ingredient of the present invention varies depending upon its type and dosage form, and also upon age, body weight or symptom of the patients, it is recommended to administer, for example, an oral preparation, to an adult about 1 to 500 mg, preferably about 3 to 300 mg per dose once to several times a day.

The drink or food of the present invention is produced by blending proanthocyanidin or the above-described preparation containing proanthocyanidin in a drink or food material during its production. For example, it is possible to make into various food forms including solid food such as bread, chewing gum, cookie, chocolate, cereal and the like, food in the form of cream or gel such as jam, ice cream, yoghurt, jelly and the like, and drink such as juice, coffee, cocoa, green tea, oolong tea, black tea and the like. Moreover, it is also possible to blend proanthocyanidin in a seasoning, a food additive or the like. As for the blending amount of proanthocyanidin or the above-described preparation containing proanthocyanidin in a drink or food material, it is not particularly limited, however, it is usually in the range from about 0 . 0001 to 80% by weight, preferably about 0.005 to 50% by weight. In addition, particularly in the case of a drink, such blending amount is in the range from 1 mg/L to 20 g/L, preferably 2 mg/L to 10g/L.

Moreover, in the case where the present invention is a drink or food, the amount of uptake of proanthocyanidin may be the same with that of the above-described oral preparation because there is no risk of the adverse effects.

The drink or food of the present invention is preferably used for body fat combustion acceleration, body fat reduction or body adiposity prevention.

Examples

Hereinafter, the present invention will be explained in detail byway of Examples exemplifying an embodiment of an extract derived from the bark of pine tree abundantly containing OPC, however, the present invention is not limited by these Examples.

Example 1

As a sample tablet, there is prepared a tablet (250 mg per tablet; proanthocyanidin content of 9 . 5 mg) wherein 8 parts by weight of an extract derived from the bark of pine tree in the seaside of France containing 40% by weight or more of proanthocyanidin (20% by weight or more as OPC) and 5% by weight or more of catechin; 22 parts by weight of crystalline cellulose; 66 parts by weight of lactose; 3 parts by weight of sucrose ester; and one part by weight of silicon dioxide are blended.

Comparative Example 1

Except that the extract derived from the bark of pine tree in the seaside of France was not used, a tablet as a placebo tablet (250 mg per tablet) was prepared similarly to Example 1.

Test Example 1

A double blind crossover test was carried out in such a manner that the sample tablets prepared in Example 1 or the placebo tablets prepared in Comparative Example 1 were administered to 9 male adults (22.1 ± 1.1 years old) as subjects for a week, and then a fixed loading exercise was performed.

After the wash out period of one week, the tablets which had not been administered last time was in turn administered to the subjects for one week, and the fixed loading exercise was performed again. Meal was adjusted from the day 3 before the fixed loading exercise so that the ratio of protein + lipid: carbohydrate equals to 1:1.

As for the administration of proanthocyanidin, the dose of proanthocyanidin was 2 tablets/day(19 mg/day) once a day, regardless of body weight of all the subjects. As for the fixed loading exercise, an exercise loading test of 20 watts every four minutes which had been gradually increased was previously carried out. An exercise loading corresponding to 2 mM of blood lactic acid concentration was calculated, and a fixed loading exercise intensity per each subject was determined. Such fixed loading exercise was performed for 60 minutes.

The subjects were laid under rest conditions from 30 minutes before the initiation of the fixed loading exercise, the expired air was collected 15 minutes before the initiation of exercise, just before exercise (0 minute), 15 minutes after the initiation of exercise, 30 minutes after that, 45 minutes after that, at the time when the exercise was terminated (60 minutes) and 15 minutes after the exercise was terminated (75 minutes), respectively. The oxygen intake (mL/min) ($VO_2$) and carbon dioxide generation amount (mL/min) ($VCO_2$) of the expired air collected at the respective time were measured by a total expired gas analyzing device using mass spectrometric analysis method (ARCO-1000 series, made by Arco System, Co. , Ltd.), and $VO_2/VCO_2$ was calculated at the respective time and expressed as a respiratory quotient (RQ).

The total amount of consumption, carbohydrate consumption calories and lipid consumption calories were calculated by the following method using the measured values:

As for the consumption calories per unit of time, it was calculated by the equation (Equation 1) of Zuntz Schmburg-Lusk.

As for the total consumption calories, it was calculated by multiplying the average values of 4 points , those are 15 minutes after the initiation of exercise, 30 minutes after that, 45 minutes after that, and the time of the exercise termination (60 minutes) out of the consumption calories per the obtained unit time, by 60 minutes.

As for carbohydrate consumption calories, after the ratio of carbohydrate consumption calories was obtained using the following equation 2 (see New ESKKA 21 Series 16, New ESKKA 21 Exercise physiology, published by Dobunshoin, p.26), the total consumption calories was calculated through integration.

As for the lipid consumption calories, it was calculated by subtracting the carbohydrate consumption calories from the total consumption calories.

The calculated results of the total consumption calories, carbohydrate consumption calories and the lipid consumption calories are shown in Fig. 1 and Table 1.

**(Equation 1) Equation of Zuntz Schmburg-Lusk**

**Consumption calories per unit time (kcal/min) = VO₂ (mL/min) × (30817+1.23×RQ) / 1000**

**(Equation 2) Equation of ratio of carbohydrate consumption calories**

**Ratio (%) of carbohydrate consumption calories = 341.67 × RQ - 240.52**

Table 1

| Taken tablet | Total consumption calories | | Carbohydrate consumption calories | | Lipid consumption calories | |
|---|---|---|---|---|---|---|
| | Sample tablet | Placebo tablet | Sample tablet | Placebo tablet | Sample tablet | Placebo tablet |
| Subject A | 732 | 658 | 560 | 514 | 172 | 144 |
| Subject B | 382 | 357 | 215 | 249 | 167 | 108 |
| Subject C | 717 | 700 | 531 | 601 | 186 | 99 |
| Subject D | 405 | 429 | 84 | 155 | 321 | 274 |
| Subject E | 656 | 642 | 507 | 523 | 149 | 119 |
| Subject F | 519 | 600 | 291 | 317 | 228 | 283 |
| Subject G | 418 | 445 | 299 | 311 | 118 | 134 |
| Subject H | 519 | 557 | 259 | 368 | 260 | 189 |
| Subject I | 519 | 455 | 282 | 362 | 237 | 93 |
| Average | 541 | 538 | 337 | 378 | 204 | 160 |
| Standard deviation | 133 | 120 | 161 | 143 | 63 | 73 |
| Standard error | 44 | 40 | 54 | 48 | 21 | 24 |

As shown in Fig. 1 and Table 1, there was no differences between the value at the time when the sample tablet was taken and the value at the time when the placebo tablet was taken concerning with the consumption calories before the exercise. When the accumulated consumption calories from the time of exercise initiation to the time of exercise termination was compared between the value at the time when the sample tablet was taken and the value at the time when the placebo tablet was taken, no difference was observed in the total consumption calories. However, when the consumption calories between carbohydrates and lipids were compared, it was confirmed that the consumption calories of carbohydrates were significantly lower and that of lipids was significantly higher at the time of administration of the sample tablet compared to at the time of administration of the placebo tablet. From these results, it is understood that proanthocyanidinhas an accelerating action on lipid metabolism.

Industrial Applicability

The present invention can provide a novel fat combustion accelerator, a body fat reducer, an adiposity preventing agent, and a drink or food for fat combustion acceleration, body fat reduction or adiposity prevention, which accelerate the lipid metabolism.

**Claims**

1.  A fat combustion accelerator, which comprises proanthocyanidin as an active ingredient.

2.  The fat combustion accelerator according to claim 1, wherein proanthocyanidin is an extract derived from pine bark .

3.  The fat combustion accelerator according to either claim 1 or 2, wherein proanthocyanidin is an oligomeric proanthocyanidin.

4.  The fat combustion accelerator according to any one of claims 1 to 3, which is in the form of tablet, pill, capsule, granule, powder, pulvis, or liquid and solution.

5.  A body fat reducer, which comprises proanthocyanidin as an active ingredient.

**EP 1 767 203 A1**

6. The body fat reducer according to claim 5, wherein proanthocyanidin is an extract derived from pine bark.

7. The body fat reducer according to either claim 5 or 6, wherein proanthocyanidin is an oligomeric proanthocyanidin.

8. The body fat reducer according to any one of claims 5 to 7, which is in the form of tablet, pill, capsule, granule, powder, pulvis, or liquid and solution.

9. An adiposity preventing agent, which comprises proanthocyanidin as an active ingredient.

10. The adiposity preventing agent according to claim 9, wherein proanthocyanidin is an extract derived from park bark.

11. The adiposity preventing agent according to either claim 9 or 10, wherein proanthocyanidin is an oligomeric proanthocyanidin.

12. The adiposity preventing agent according to any one of claims 9 to 11, which is in the form of tablet, pill, capsule, granule, powder, pulvis, or liquid and solution.

13. A drink or food, which comprises the fat combustion accelerator according to any one of claims 1 to 4.

14. A drink or food, which comprises the body fat reducer according to any one of claims 5 to 8.

15. A drink or food, which comprises the adiposity preventing agent according to any one of claims 9 to 12.

16. A drink or food for fat combustion acceleration, body fat reduction or adiposity prevention, which comprises proanthocyanidin as an active ingredient.

17. The drink or food according to claim 16, which is a drink or food for body fat combustion acceleration.

18. The drink or food according to claim 16, which is a drink or food for body fat reduction.

19. The drink or food according to claim 16, which is a drink or food for body adiposity prevention.

20. The drink or food according to any one of claims 16 to 19, wherein proanthocyanidin is an extract derived from pine bark.

21. The drink or food according to any one of claims 16 to 20, wherein proanthocyanidin is an oligomeric proanthocyanidin.

22. The drink or food according to any one of claims 16 to 21, which is a solid food, a gel food, or a drink.

23. The drink or food according to any one of claims 16 to 22, which is a soft drink or a tea drink.

Fig. 1

## INTERNATIONAL SEARCH REPORT

| International application No. |
| --- |
| PCT/JP2005/011904 |

**A. CLASSIFICATION OF SUBJECT MATTER**
*A61K31/352* (2006.01), *A23F3/14* (2006.01), *A23L1/30* (2006.01),
*A61K36/00* (2006.01), *A61P3/06* (2006.01)

According to International Patent Classification (IPC) or to both national classification and IPC

**B. FIELDS SEARCHED**

Minimum documentation searched (classification system followed by classification symbols)
*A61K31/352* (2006.01), *A23F3/14* (2006.01), *A23L1/30* (2006.01),
*A61K36/00* (2006.01), *A61P3/06* (2006.01)

Documentation searched other than minimum documentation to the extent that such documents are included in the fields searched
Jitsuyo Shinan Koho          1922-1996     Jitsuyo Shinan Toroku Koho     1996-2005
Kokai Jitsuyo Shinan Koho    1971-2005     Toroku Jitsuyo Shinan Koho     1994-2005

Electronic data base consulted during the international search (name of data base and, where practicable, search terms used)
FSTA(STN), CAplus(STN), MEDLINE(STN), JMEDPlus(JOIS), JSTPlus(JOIS)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2003-146898 A (Kabushiki Kaisha Toyo Shin'yaku), 21 May, 2003 (21.05.03), Full text; particularly, Claims 2, 5; examples (Family: none) | 1-23 |
| X | JP 61-16982 A (Kikkoman Corp.), 24 January, 1986 (24.01.86), Examples & US 4797421 A | 13-22 |
| X | JP 8-225453 A (Suntory Ltd.), 03 September, 1996 (03.09.96), Claim 8; examples & EP 713706 A2 | 13-23 |

|☒| Further documents are listed in the continuation of Box C. | ☐ See patent family annex. |

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| 30 September, 2005 (30.09.05) | 18 October, 2005 (18.10.05) |

| Name and mailing address of the ISA/ | Authorized officer |
| --- | --- |
| Japanese Patent Office | |
| Facsimile No. | Telephone No. |

Form PCT/ISA/210 (second sheet) (January 2004)

**INTERNATIONAL SEARCH REPORT**

International application No.

PCT/JP2005/011904

C (Continuation). DOCUMENTS CONSIDERED TO BE RELEVANT

| Category* | Citation of document, with indication, where appropriate, of the relevant passages | Relevant to claim No. |
|---|---|---|
| X | JP 2001-64172 A (Kikkoman Corp.),<br>13 March, 2001 (13.03.01),<br>Claims 2, 5; examples; Par. No. [0021]<br>(Family: none) | 13-23 |
| X | Teruo SENSO et al., "Meta-analysis ni yoru Catechin-rui no Taishibo Teigen Koka no Kensho", The Japanese Society of Nutrition and Food Science Sokai Koen Yoshishu, Vol.58, page 187 (2004 Apr.) | 1-23 |
| X | WO 2004/010991 A1 (Kabushiki Kaisha Toyo Shin'yaku),<br>05 February, 2004 (05.02.04),<br>Full text; particularly, Claims<br>& EP 1547591 A1 | 13-23 |
| P,X | JP 2004-242663 A (Kabushiki Kaisha Toyo Shin'yaku),<br>02 September, 2004 (02.09.04),<br>Full text<br>(Family: none) | 1-23 |
| P,X | JP 2005-47839 A (Kabushiki Kaisha Toyo Shin'yaku),<br>24 February, 2005 (24.02.05),<br>Full text<br>(Family: none) | 1-23 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3007232 B

- JP 19917232 B

**Non-patent literature cited in the description**

- **BAGCHI, D. et al.** *Toxicology,* 2000, vol. 148, 187-189
- **FREMONT L. et al.** *Life Sciences,* 1999, vol. 64, 2511-2521

- **R. W. HEMINGWAY et al.** *Phytochemistry,* 1983, vol. 22, 275-281